# EUROPÄISCHE PATENTANMELDUNG

(11) **EP 0 870 781 A1**
(43) Veröffentlichungstag der Anmeldung: **14.10.1998**
(21) Anmeldenummer: 98104772.3
(22) Anmeldetag: 17.03.1998
(51) Int. Cl.: C08F 20/34, C08F 26/02, C08F 293/00, C08G 65/00, C02F 1/56

(54) **Blockcopolymere und deren Verwendung als polymere Tenside**

(30) Priorität: 09.04.1997 DE 19714714
(71) Anmelder: BASF AKTIENGESELLSCHAFT, 67056 Ludwigshafen (DE)
(72) Erfinder: Sanner, Axel, Dr., 67227 Frankenthal (DE); Zeitz, Katrin, Dr., 67067 Ludwigshafen (DE); Lieske, Antje, 10317 Berlin (DE); Jaeger, Werner, Dr., 14532 Kleinmachnow (DE)

(57) **Zusammenfassung**

Blockcopolymere der Struktur A-B-A, in der A einen Polyethylenoxid-Block darstellt und B ein Polymerisat aus olefinisch ungesättigten quaternären Ammoniumverbindungen. Die Blockcopolymere eignen sich zur Verwendung als polymere Tenside.

## Beschreibung

Die vorliegende Erfindung betrifft neue Blockcopolymere der Struktur A-B-A, in der A einen Polyethylenoxid-Block darstellt und B ein Polymerisat aus olefinisch ungesättigten quaternären Ammoniumverbindungen. Weiterhin betrifft die Erfindung ein Verfahren zu deren Herstellung und deren Verwendung als polymere Tenside.

Polymere Tenside werden in wäßrigen Systemen zur Stabilisierung von Emulsionen, als tensidische Komponente in Emulsionspolymerisationen und zur Solubilisierung wasserunlöslicher Stoffe eingesetzt. Entsprechend ihrer chemischen Struktur können die polymeren Tenside in amphiphile Blockcopolymere und Polyseifen unterschieden werden.

Den Hauptanteil der praktisch genutzten Tenside stellen die amphiphilen Blockcopolymere, insbesondere Blockcopolymere mit Polyethylenoxid- und Polypropylenoxidblöcken dar (L.G. Lundsted, I.R. Schmolka in "Block and Graft Copolymerisation", Hrsg, R.J. Ceresa Wiley, London 1976, Vol. 2, S 113 ff; I.R. Schmolka J. Am. Oil Chem. Soc. 54 (1977) 110; I. Piirma, Macromol Chem., Macromol Symp. 35/36 (1990) 467).

Sie bieten den Vorteil, daß der HLB-Wert dieser Verbindungen durch Variation der Struktur und der Länge der hydrophilen bzw. hydrophoben Blöcke dem gewünschten Anwendungszweck angepaßt werden kann. Nachteilig wirkt sich aus, daß die amphiphilen Blockcopolymere wie niedermolekulare Tenside intermolekular aggregieren und somit ihre Wirksamkeit an Konzentrationen oberhalb ihrer kritischen Mizellbildungskonzentration gebunden ist. Mit diesen Verbindungen stabilisierte Latices und Emulsionen sind daher nur eingeschränkt verdünnungsstabil. Solubilisierte Stoffe werden bei Verdünnung des Systems desolubilisiert. Von Nachteil ist weiterhin, daß für die Gewinnung der amphiphilen Blockcopolymere aufwendige Syntheseverfahren wie z.B. die lebende anionische Polymerisation oder quasi-lebende kationische Polymerisationen erforderlich sind. Eine direkte Synthese von amphiphilen Blockcopolymeren, die Blöcke mit ionischer Ladung enthalten, ist auf diesen Wegen nicht möglich. Derartige Polymere sind nur durch einen zusätzlichen, in der Regel unvollständig verlaufenden Verfahrensschritt zugänglich, nämlich durch nachgeschaltete Funktionalisierung von Blockcopolymeren (W.J. Choi, Y.B. Kim, S.K. Kwon, K.T. Lim, S.K. Choi, J. Polymer Sci. A, Polymer Chem. 30 (1992) 2143).

Polymere Seifen sind durch radikalische Polymerisation von tensidischen Monomeren oder aber durch Modifizierung eines hydrophilen Polymeren mit hydrophoben Resten und umgekehrt zugänglich.

Sie bilden Mizellen von kleinen Konzentrationen an und eröffnen damit die Möglichkeit zur Herstellung verdünnungsstabiler Emulsionen und Latices und zur Reduzierung der erforderlichen Tensidmenge (A. Laschewski; Adv. Polym. Sci 124 (1995) 1; Y.J. Yang, J.B.F.N. Engberts; Eur. Polym. J. 28 (1992) 881). Von Nachteil ist allerdings, daß die Einstellung ausgewogener Verhältnisse von hydrophilen und hydrophoben Eigenschaften nur sehr schwierig zu realisieren ist. Eine klare Trennung der hydrophilen und der hydrophoben Teile des Moleküls wie bei den amphiphilen Blockcopolymeren ist bei den polymeren Seifen nicht möglich.

Aufgabe der vorliegenden Erfindung war es, neue polymere Tenside mit verbesserten Eigenschaften zu finden.

Demgemäß wurden die eingangs definierten Blockcopolymere, ein Verfahren zu ihrer Herstellung und ihre Verwendung gefunden.

Nach dem erfindungsgemäßen Verfahren erfolgt die Herstellung der Blockcopolymere durch radikalische Polymerisation der olefinisch ungesättigten quaternären Ammoniumverbindungen in Gegenwart eines Makroinitiators. Als Lösungsmittel wird Wasser eingesetzt. Die Polymerisationstemperatur liegt im Bereich von 40 bis 95°C, vorzugsweise 55 bis 80°C.

Als Makroinitiator wird erfindungsgemäß ein Umsetzungsprodukt aus einem Azostarter und einem Polyethylenglykol mit einem mittleren Polymerisationsgrad m eingesetzt. Als Azostarter wird vorzugsweise Azo-bis-isobutyronitril eingesetzt. Die Polyethylenglykolkomponente weist einen mittleren Polymerisationsgrad m auf, wobei m für eine ganze Zahl von 5 bis 100 steht. Der resultierende Makroinitiator entspricht vorzugsweise der allgemeinen Formel IV

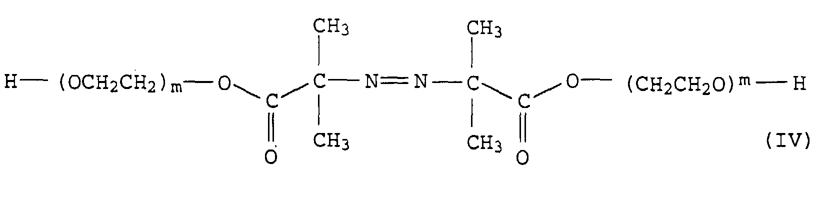

Die Synthese der Makroinitiatoren erfolgt nach an sich bekannten Verfahren (R. Walz et al., Makromolekulare Chemie, 178 (1977) 2527).

Unter Polymerisationsbedingungen erfolgt wie für Azostarter üblich die Bildung von Radikalen unter Stickstoffabspaltung. Der Polyethylenoxidblock A, der aus einer Kette von m Ethylenoxid-Einheiten und einem niedermolekularen Verknüpfungsglied -O-CO-C(CH₃)₂- besteht, wird also über den Initiator in das Blockcopolymer eingeführt.

Der Aufbau einer Blockstruktur A-B-A kann durch Zugabe entsprechender Mengen an Initiator gesteuert werden. Es empfiehlt sich, den Initiator in Mengen von 1x10⁻² mol/l bis 2x10⁻² mol/l, bezogen auf die Ausgangsmonomerlösung, einzusetzen.

Als Monomere zum Aufbau des kationischen Seifenblocks B eignen sich erfindungsgemäß die Monomeren I der allgemeinen Formel I 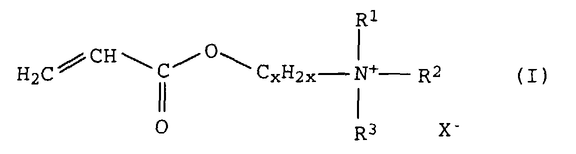 in der x für eine Zahl von 5 bis 18, vorzugsweise 8 bis 14 steht, R¹ und R² unabhängig voneinander einen Alkylrest mit 1 bis 5 C-Atomen, vorzugsweise 1 bis 3 C-Atomen, R³ für ein Alkyl oder Hydroxyalkyl mit 1 bis 5, vorzugsweise 1 bis 3 C-Atome, stehen und X⁻ ein Halogenid, Acetat oder Methosulfat, vorzugsweise Chlorid oder Bromid, bedeutet.

Weiterhin eignen sich als Monomere für den Block B Mischungen der Monomeren II und III der allgemeinen Formeln II und III 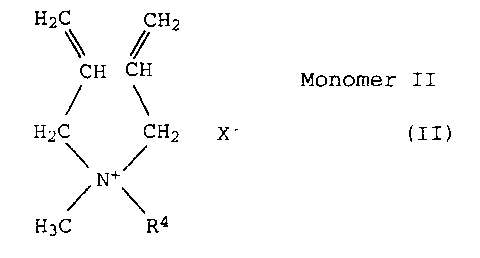 in der R⁴ für einen Rest CₙH₂ₙ₊₁ steht und n eine Zahl von 5 bis 18, vorzugsweise 8 bis 14 bedeutet,
und 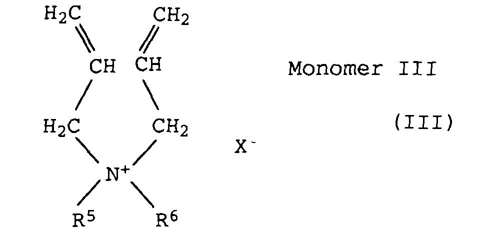 in der R⁵ und R⁶ für einen Alkyl- oder Hydroxyalkylrest mit 1 bis 3 C-Atomen, vorzugsweise einen Methylrest, stehen,
und X⁻ für die Monomere II und III ein Halogenid, vorzugsweise Chlorid, Acetat oder Methosulfat bedeutet.

Die Mengenverhältnisse für die Monomeren I oder die Mischungen der Monomeren II und III werden so gewählt, daß der resultierende Block B aus z Einheiten der Monomeren I, wobei z eine Zahl von 10 bis 200 bedeutet, oder aus p Einheiten der Monomeren II und q Einheiten der Monomeren III, wobei p und q unabhängig voneinander jeweils eine Zahl von 5 bis 100 darstellen, aufgebaut ist. Das Verhältnis der Monomeren II zu den Monomeren III beträgt 1:20 bis 20:1.

Die Herstellung der Monomeren I, II und III kann nach bekannten Verfahren erfolgen (siehe: Y.J. Yang, J.B.F.N. Egberts; J. Org. Chem. 56, 4300 (1991); Houben-Weyl "Methoden der organischen Chemie" Georg Thieme Verlag, Stuttgart 1958, Bd. 11/2, S. 597; D.F. Evans, H. Wennerström "The Colloidal domain", VCH Publishers, INc.; New York, 1994, S. 6).

Das Verhältnis des Polyethylenoxid-Blocks mit m Einheiten zu dem kationischen Seifenblock kann durch geeignete Wahl der Mengen von Ausgangsstoffen beliebig eingestellt werden. Vorzugsweise beträgt das Verhältnis m zu z beziehungsweise m zu (p+q) 2:1 bis 1:2.

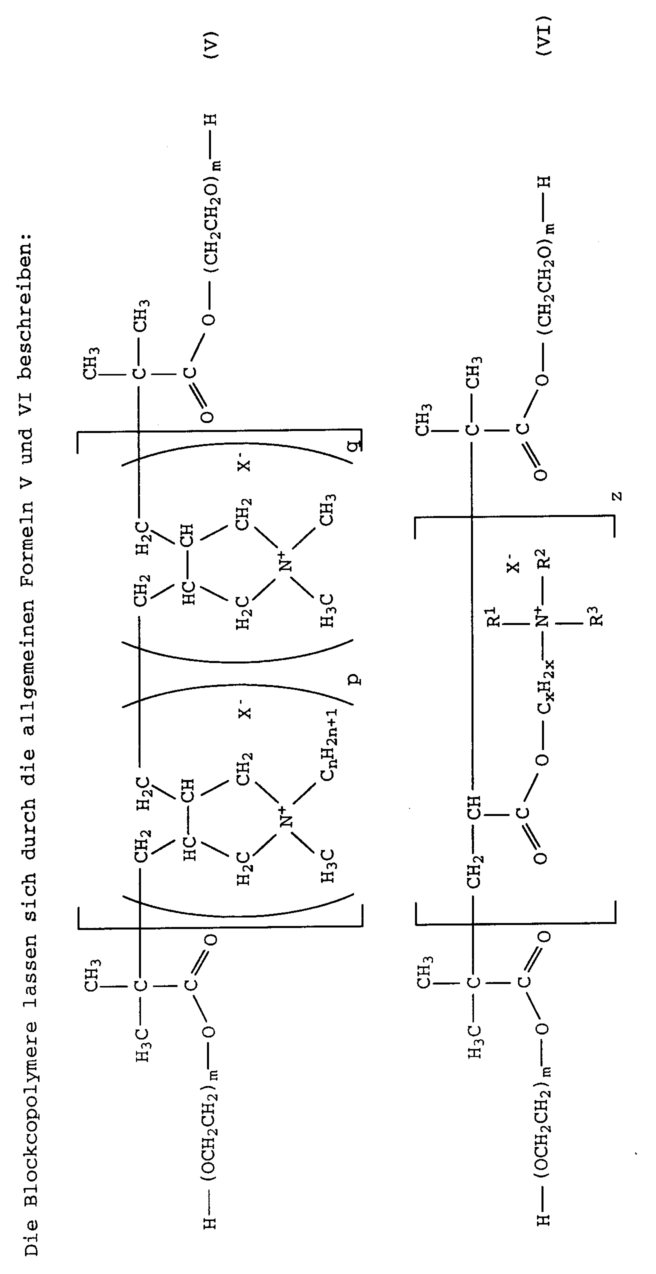

Die resultierenden Blockcopolymere weisen im kationischen Polyseifen-Block B Molmassen im Bereich von 1000 bis 350000 g/mol und im Polyethylenoxid-Block Molmassen im Bereich von 200 bis 30000 g/mol auf.

Die erfindungsgemäßen Blockcopolymere weisen im Vergleich zu bisher bekannten polymeren Tensiden deutlich verbesserte Eigenschaften hinsichtlich der Solubilisierungskapazität bei gleichzeitig guter Verdünnungsstabilität der entsprechenden stabilisierten Latices, Emulsionen, Solubilisate etc. auf.

Das erfindungsgemäße Verfahren erlaubt auf einfache Weise die gezielte Einstellung der oberflächenaktiven Eigenschaften des Blockcopolymers.

Die Blockcopolymere eignen sich als polymere Tenside zur Stabilisierung oder Solubilisierung von in Wasser unlöslichen oder schwerlöslichen biologisch aktiven Substanzen in wäßrigen Formulierungen, ebenso wie als Solubilisierungshilfsmittel oder Netzmittel in festen Formulierungen. Biologisch aktive Substanzen in diesem Sinne sind pharmazeutische oder veterinärmedizinische Wirkstoffe, Pflanzenschutzmittel oder kosmetische Wirkstoffe. Entsprechende Formulierungen sind beispielsweise kosmetische Formulierungen wie Emulsionen oder Lotionen für die Hautpflege oder Haarpflegepräparate wie beispielsweise Schäume.

Weiterhin eignen sich die Blockcopolymere zur Verwendung in flüssigen oral zu verabreichenden pharmazeutischen Zubereitungen oder insbesondere für intravenös zu verabreichende Formen zur Verbesserung der Bioverfügbarkeit oder auch für topisch anzuwendende pharmazeutische Formulierungen, insbesondere Emulsionen, Lotionen oder Suspensionen.

Die erfindungsgemäßen Blockpolymere eignen sich weiterhin als polymere Tenside zur Herstellung stabiler flüssiger Formulierungen von Carotinoiden wie beispielsweise β-Carotin oder Astaxanthin zur Anwendung in Nahrungsmitteln oder in der Tierernährung.

### Herstellung von Blockcopolymeren

### Allgemeine Vorschrift

Die Herstellung der Blockcopolymere gemäß den nachstehenden Beispielen 1 bis 3 erfolgte in einem thermostatisierbaren, innentemperaturgeregelten Doppelmantelreaktor mit Rührer, Rückflußkühler, Temperaturfühler und Gaseinleitungsrohr. Die Ausgangsmischung aus Monomeren und Wasser wurde in den Reaktor eingebracht, eine Stunde mit Stickstoff gespült und dann auf die Reaktionstemperatur von 65°C erwärmt. Nach Erreichen der Reaktionstemperatur wurde der Makroinitiator in einer Probe der Ausgangsmischung gelöst und in die Reaktionsmischung gegeben. Nach einer Reaktionszeit von 6 Stunden wurde die Mischung auf Eis gegeben und unter Verwendung einer Membran vom Typ Open Channel Omega der Fa. Filtron mit einer Ausschlußgrenze von 5000 Dalton ultrafiltriert, bis das Filtrat eine Restleitfähigkeit von 10 µS aufwies. Im Falle der Beispiele 1 und 2 wurde das Retentat gefriergetrocknet, gemäß Beispiel 3 eingeengt und das Polymer mit einem 10-fachen Überschuß an Aceton ausgefällt.

Die Mengen an jeweils verwendeten Einsatzstoffen sind unter den Beispielen aufgeführt.

### Beispiel 1

### Ausgangsmischung:

13,0 g Monomer II mit R⁴ = n-Octyl und X⁻ = Chlorid und 18,1 g einer 67 gew.-%igen wäßrigen Diallyldimethylammoniumchloridlösung (Monomer III) mit Wasser auf 50 ml aufgefüllt.

### Makroinitiatorlösung:

2,08 g eines Umsetzungsproduktes aus Azo-bis-isobutyronitril und Polyethylenglykol (m = 45), gelöst in 5 ml Ausgangsmischung.

Es wurden 8,1 g eines Blockcopolymeren mit mittleren Werten von m = 45, p = 35 und q = 55 mit einem Verhältnis von m:(p+q)= 1:2 erhalten.

Die Solubilisierungskapazität des Blockcopolymers in Bezug auf n-Dekanol war um einen Faktor von 5,1 größer als die Solubilisierungskapazität einer entsprechenden nur aus B bestehenden reinen Polyseife. Ein analoges Blockcopolymer mit einem ausschließlich aus Monomer III bestehenden Block B war nicht in der Lage, Dekanol zu solubilisieren.

### Beispiel 2

### Ausgangsmischung:

12,4 g Monomer II mit R⁴ = n-C₁₂H₂₅ und X⁻ = Chlorid und 16,2 g einer 67 gew.-%igen wäßrigen Lösung von Diallyldimethylammoniumchlorid mit Wasser auf 50 ml aufgefüllt.

### Makroinitiatorlösung:

analog Beispiel 1

Es wurden 6,4 g eines Blockcopolymers mit mittleren Werten m = 45, p = 5 und q = 33 mit einem Verhältnis von m:(p+q)= 1,18:1 erhalten.

Die Solubilisierungskapazität für n-Dekanol war um einen Faktor von 1,8 größer als die der entsprechenden reinen Polyseife.

### Beispiel 3

### Ausgangsmischung:

15,8 g Monomer I mit x = 11 und X⁻ = Bromid in 400 ml Wasser.

### Makroinitiatorlösung:

16,66 g des Umsetzungsproduktes gemäß Beispiel 1 in 30 ml Wasser

Es wurden 13,8 g Blockcopolymer mit mittleren Werten m = 45 und z = 75 mit einem Verhältnis m:z = 1:1,7 erhalten.

Die Solubilisierungskapazität für n-Dekanol war um einen Faktor 1,35 größer als die der entsprechenden reinen Polyseife.

## Patentansprüche

1. Blockcopolymere der Struktur A-B-A, in der A einen Polyethylenoxid-Block darstellt und B ein Polymerisat aus olefinisch ungesättigten quaternären Ammoniumverbindungen.

2. Blockcopolymere nach Anspruch 1, enthaltend einen Block A mit einem mittleren Polymerisationsgrad m von 5 bis 100.

3. Blockcopolymere nach Anspruch 1 oder 2, enthaltend als Block B ein Polymerisat aus quaternären Ammoniumverbindungen (Monomere I) der allgemeinen Formel I 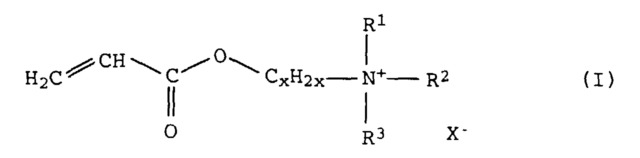 in der x eine Zahl von 5 bis 18, R¹, R² unabhängig voneinander einen C₁-C₅-Alkylrest und R³ einen C₁-C₅-Alkyl- oder Hydroxyalkylrest und X⁻ Halogenid, Acetat oder Methosulfat bedeuten.

4. Blockcopolymere nach Anspruch 1 oder 2, enthaltend als Block B ein Polymerisat aus Mischungen der Monomere II der allgemeinen Formel II 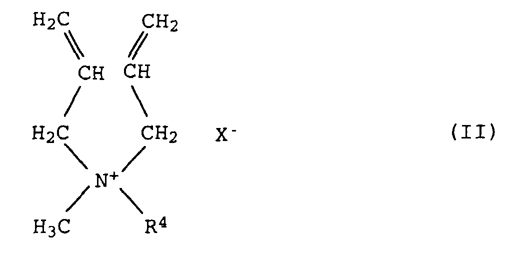 in der R⁴ für einen Rest CₙH₂ₙ₊₁ steht und n eine Zahl von 5 bis 18 ist,
und der Monomere III der allgemeinen Formel III 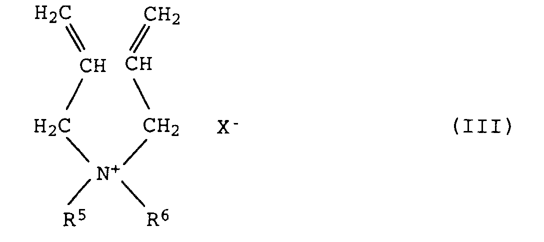 in der R⁵ und R⁶ für einen C₁-C₃-Alkylrest oder einen C₁-C₃-Hydroxyalkylrest stehen,
und X⁻ für die Monomere II und III Halogenid, Acetat oder Methosulfat bedeutet, wobei das Verhältnis der Monomere II zu den Monomeren III 1:20 bis 20:1 beträgt.

5. Blockcopolymere nach einem der Ansprüche 1 bis 4, enthaltend einen Block B der Molmasse 1000 bis 350000 g/mol.

6. Blockcopolymere nach einem der Ansprüche 1 bis 5, enthaltend Polyethylenoxid-Blöcke A der Molmasse 200 bis 30000 g/mol.

7. Verfahren zur Herstellung von Blockcopolymeren gemäß einem der Ansprüche 1 bis 6, durch radikalische Polymerisation olefinisch ungesättigter quaternärer Ammoniumverbindungen in wäßriger Lösung in Gegenwart eines Makroinitiators, der durch Umsetzung von Polyethylenglykol mit Azo-bis-isobutyronitril erhalten wird.

8. Verwendung der Blockcopolymere gemäß einem der Ansprüche 1 bis 6 als polymere Tenside zur Herstellung von Formulierungen biologisch aktiver Substanzen.

9. Verwendung nach Anspruch 8 zur Herstellung pharmazeutischer Formulierungen.

10. Verwendung nach Anspruch 8 zur Herstellung kosmetischer Formulierungen.
